# Europäisches Patentamt

(19) **European Patent Office**

**Office européen des brevets**

(11) Numéro de publication: **0 128 784**
**B1**

(12) **FASCICULE DE BREVET EUROPEEN**

(45) Date de publication du fascicule du brevet:
**28.12.88**

(51) Int. Cl.⁴: **A 61 F 2/16**

(21) Numéro de dépôt: **84400601.5**

(22) Date de dépôt: **26.03.84**

(54) Implant oculaire de chambre postérieure.

---

(30) Priorité: **16.05.83 FR 8308037**

(43) Date de publication de la demande:
**19.12.84 Bulletin 84/51**

(45) Mention de la délivrance du brevet:
**28.12.88 Bulletin 88/52**

(84) Etats contractants désignés:
**AT BE CH DE FR GB IT LI LU NL SE**

(56) Documents cités:
**WO-A-83/01568**
**WO-A-83/03753**
**WO-A-84/00883**
**DE-U- 8 336 372**
**FR-A- 2 515 956**
**GB-A- 2 050 173**
**US-A- 3 991 426**
**US-A- 4 092 743**
**US-A- 4 244 060**

(73) Titulaire: **Aron-Rosa, Danièle Sylvie, 28 avenue Raphael, F-75016 Paris (FR)**

(72) Inventeur: **Aron-Rosa, Danièle Sylvie, 28 avenue Raphael, F-75016 Paris (FR)**

(74) Mandataire: **Viard, Jean, Cabinet VIARD 28 bis, avenue Mozart, F-75016 Paris (FR)**

---

ACTORUM AG

## Description

La présente invention a pour objet un cristallin artificiel ou implant intraoculaire de chambre postérieure destiné en particulier, mais non exclusivement, à être mis en place après une opération de la cataracte. Le but de cette prothèse est d'éviter à l'opéré le port permanent de lunettes très épaisses destinées à pallier l'absence du cristallin naturel.

Le cristallin naturel chez l'homme est une structure transparente dont le diamètre est de l'ordre de 9 mm pour une épaisseur de 5 mm environ, de forme générale lenticulaire, suspendue derrière l'iris par des fibres zonulaires qui relient le cristallin au corps ciliaire. La cataracte consiste en une opacification du cristallin ou des capsules, antérieure ou postérieure qui constituent le sac cristallin. L'opération de la cataracte consiste en l'ablation du cristallin et est désignée comme «intracapsulaire» lorsque la capsule est retirée en même temps que le cristallin et, comme «extracapsulaire» lorsque la capsule antérieure est extraite avec le cristallin alors que la capsule postérieure est laissée à l'intérieur de l'œil.

La première lentille intraoculaire a été implantée par RIDLEY en 1949 et, depuis cette date, différents types de cristallins artificiels ont été proposés. On peut citer, notamment, les brevets US-A-3 991 426 et US-A-4 092 743.

Parmi les problèmes que posent l'implantation et le port de lentilles intraoculaires celui de la fixation de l'implant à l'intérieur de la cavité oculaire est l'un des plus critiques. Pour des raisons de tolérance, les lentilles, qui étaient à l'origine disposées dans le plan irien, sont maintenant implantées soit dans la chambre antérieure constituée par la cornée et l'iris, soit dans la chambre postérieure constituée par l'iris et la capsule postérieure du cristallin située en avant de la membrane hyaloïde de l'humeur vitrée. La présente invention concerne plus particulièrement les implants de chambre postérieure positionnés après une opération de la cataracte extracapsulaire. Les implants sont maintenus par des anses souples prenant appui, dans le cas d'un implant de chambre postérieure, par leurs extrémités libres sur le sulcus ciliaire (ou corps ciliaire) ou dans le sac du cristallin. Toutefois, la capsule postérieure peut elle-même s'opacifier quelques années après l'opération de la cataracte. Il est alors nécessaire de réouvrir l'œil malade.

Pour permettre cette seconde opération, il a été proposé, dans le Brevet US-A-4 244 060 de prévoir, sur la face postérieure d'un implant, une lèvre annulaire comprenant une ouverture. Il est ainsi possible, bien que délicat, de passer un instrument tranchant à travers cette ouverture pour ouvrir la capsule postérieure opacifiée sans retirer l'implant. Malheureusement, la capsule postérieure a tendance à s'invaginer à l'intérieur de l'anneau contre la face postérieure de l'implant.

Par ailleurs, la Demanderesse a développé une nouvelle thérapie faisant appel à un rayon laser pulsé fonctionnant en mode bloqué qui permet, par un effet de claquage optique d'intervenir dans des régions parfaitement déterminées de l'œil sans avoir à ouvrir celui-ci. (Brevet Européen EP-0 007 256). Il est ainsi possible, avec l'appareil décrit dans ce brevet d'ouvrir une enveloppe cristalline opacifiée en une fraction de seconde, sans recourir aux moyens de la chirurgie ophtalmologique classique. Malheureusement, le traitement laser utilisé derrière un cristallin artificiel peut marquer celui-ci. En effet, la capsule prend appui sur la face postérieure de l'implant et le faisceau laser d'ouverture de la capsule peut marquer l'implant d'une façon définitive.

Un objet de la présente invention est un implant de chambre postérieure permettant un traitement par laser d'un malade portant un tel implant, qui soit aisé à fabriquer et permettre une circulation des liquides entre les chambres postérieure et antérieure.

WO 83/01568 décrit un implant de chambre postérieure constitué d'une lentille plan convexe, asphérique ou biconvexe sur laquelle sont fixées des anses courbes et souples.

DE-U-8 336 372 décrit un implant intraoculaire de chambre postérieure comprenant les caractéristiques énoncées dans le préambule de la revendication 1.

Selon l'invention, l'implant du type décrit dans DE-U-8 336 372 est caractérisé en ce que les éléments distanceurs sont constitués par des pièces tronconiques insérées dans des trous tronconiques de la lentille, ces pièces étant éloignées de 0,1 à 0,4 mm du bord de la lentille et faisant saillie à l'extérieur de la face postérieure de la lentille sur une longueur de 0,3 à 0,4 mm mesurée aux endroits où sont percés les trous, de sorte que la distance entre la capsule postérieure et la face postérieure soit au moins égale à 0,3 mm.

Il est ainsi possible de traiter la capsule malade à l'aide d'un rayon laser, sans marquer l'implant.

Pour être efficaces, les éléments doivent être éloignés de 0,1 à 0,4 mm du bord de l'implant de manière à ce que la capsule soit tendue selon deux directions perpendiculaires et suspendue sur les indentations sans que l'implant ne se déplace et sans que la capsule ne vienne s'invaginer contre la face postérieure de l'implant, c'est-à-dire adhérer à cette face. La discontinuité des éléments évite, derrière celui-ci l'accumulation de cortex cristallinien ou de liquides variés tels que, notamment l'acide hyaluronique si il a été utilisé au cours de l'intervention, et qui peut être piégé dans cet espace, malgré la présence des trous de positionnement dont on ne sait jamais si ils resteront perméables ou s'obtureront par symphise capsulaire si l'implant est dans le sac capsulaire ou par du matériel cristallinien resté, durant l'intervention de la cataracte, dans l'espace laissé libre entre le bord de l'implant et les saillies d'indentation. L'implant selon l'invention permet de créer un espace librement communiquant pour éviter les blocages rétropseudophaquos (Accumulation d'humeurs aqueuses, de matériel cristallinien, d'exsudats et d'acide hyaluronique éventuel).

La hauteur des indentations, de 0,3 à 0,4 mm au-dessus de la face arrière dépend de la longueur du claquage optique. Lorsque l'ophtalmologue utilise des laser à fréquence moins élevée que le laser picoseconde, l'onde de choc est plus longue. De plus, la visée proprement dite se fait, pour les laser YAG, habituellement à l'aide d'un laser Hélium-Néon auxiliaire coïncidant, le claquage YAG se produisant de 0,3 à 0,4 mm en avant du faisceau He-Ne de visée pour les énergies normalement nécessaires pour une capsulotomie soit, sensiblement, de 2 à 4 millijoules.

D'autres caractéristiques et avantages de l'invention apparaîtront au cours de la description qui va suivre de modes particuliers de réalisation de l'invention, donnés uniquement à titre d'exemples non limitatifs, en regard des dessins qui représentent:

– Les figures 1 à 3, un mode de réalisation d'un implant selon l'invention;

– La figure 4, une vue en coupe d'un œil dans lequel est implanté un cristallin artificiel selon l'invention.

Dans les exemples représentés, l'implant se compose d'une lentille plan convexe ou biconvexe, la face avant étant convexe, la face arrière pouvant être plane ou légèrement convexe. Cette structure correspond aux nécessités physiologiques de focalisation sur la rétine. Le cristallin est réalisé en matière plastique relativement souple et, par exemple en polyméthyl méthacrylate de polypropylène (PMMA) ou en «Perspex» (Marque déposée). Le cristallin se présente sous la forme d'une lentille d'au moins 6 mm de diamètre. Il est fixé dans la chambre postérieure de l'œil au moyen de deux anses insérées sur la face arrière de la lentille. Selon une caractéristique de la présente invention, les deux anses sont montées de manière à être inclinées vers l'avant d'un angle sensiblement égal à 10° par rapport au plan de la lentille, et mesurent plus de 13,5 mm de long et moins de 15 mm.

Les extrémités 4 des anses 2 sont emmanchées à force dans des cavités latérales formées dans la lentille 1. La longueur totale des anses ou haptiques est de 13 mm au minimum et de 14,5 mm au maximum.

Les deux anses peuvent être soit de type «Sinskey» (Marque déposée), soit de type«J Loop», l'anse supérieure pouvant présenter une encoche 7a (représentée en tirets) alignée avec la ligne midi-six heures passant par le trou de positionnement 6, de manière à ce qu'elle puisse être saisie et abaissée sur le trou de positionnement supérieur de l'implant à l'aide d'une pince spéciale.

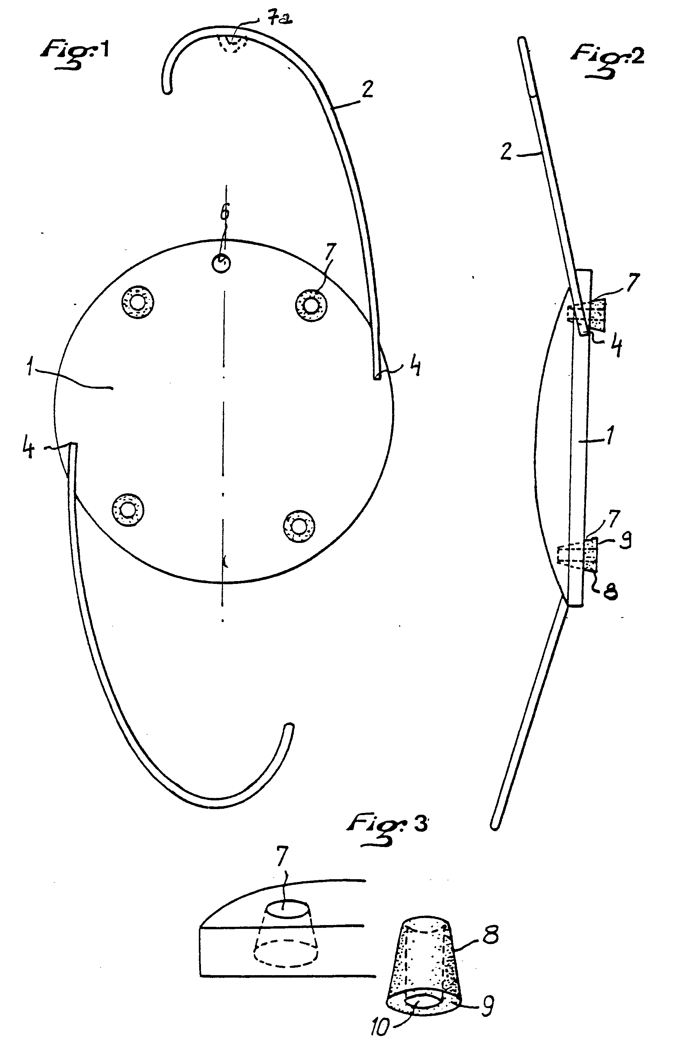

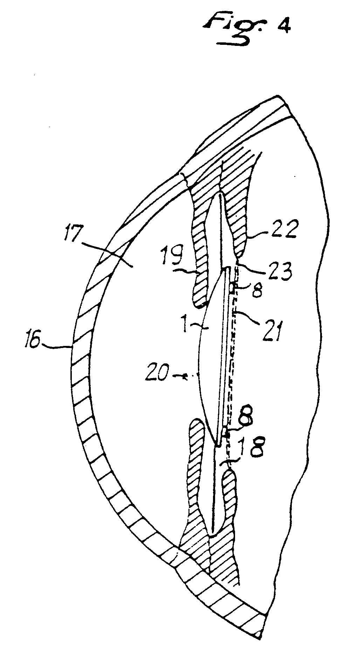

Les figures 1 à 3 représentent un mode de réalisation d'un implant selon l'invention. Pour éviter les décentrements, le diamètre de la lentille 1 est supérieur à 6 mm et, par exemple compris entre 6,5 et 7 mm. Sa face antérieure ou supérieure est convexe, alors que sa face postérieure ou inférieure est plane ou convexe. Outre le trou de positionnement 6 de midi, la lentille 1 présente quatre trous tronconiques 7 disposés en carré sensiblement dans les directions de 11 heures, 1 heure, 5 heures et 7 heures. Chaque trou 7 est dirigé dans le sens antéropostérieur, son axe étant perpendiculaire à la face postérieure. Les trous 7 ont une forme générale tronconique dont le diamètre avant est de 0,3 mm, et dont le diamètre arrière est de 0,45 à 0,65 mm.

A l'intérieur des trous tronconiques 7 sont montés des pièces également tronconiques 8 dont la hauteur est supérieure de 0,3 à 0,4 mm à l'épaisseur de la lentille aux endroits où sont percés les trous 7, les pièces tronconiques étant éloignées du bord de 0,1 mm environ. Les pièces 8 présentent une base 9 lisse. Elles sont percées d'un orifice central 10 dont le diamètre est de 0,3 mm assurant la perméabilité entre les chambres antérieure et postérieure. La conicité du trou 7 et d'une pièce 8 assure une liaison mécanique satisfaisante.

Comme cela a été mentionné précédemment, la capsule postérieure est tendue sur les indentations. Une opération par éclairs laser produit une ligne discontinue d'impacts. Avec une disposition en carré des indentations, celles-ci constituent un ensemble de repères pour une coupe de la capsule selon les lignes médianes. Ainsi, un minimum de points d'impact est nécessaire pour une ouverture parfaitement axiale, verticale ou horizontale, qui n'est pas gênante pour le passage de l'axe visuel.

Dans le cas particulier de cet implant, la longueur des anses est comprise entre 14 et 14,5 mm, hors tout, de manière à éviter les excentrements de l'implant. Les anses forment, avec le plan de la surface postérieure un angle de 10° vers l'avant. Cette inclinaison a pour but d'éviter que l'implant ne vienne porter sur la face postérieure de l'iris même si l'implant repose sur le sulcus ciliaire. Cette caractéristique évite également les captures iriennes. L'anse présente une encoche 7a, de manière à ce qu'il soit possible de procéder à une refixation en cas de luxation de l'implant.

Le trou de midi 6 est un trou de saisie de l'implant en vue de son positionnement en chambre postérieure.

On a représenté sur la figure 4, un implant selon l'invention en position à l'intérieur d'un œil. On distingue sur cette figure, la cornée transparente 16, la chambre antérieure 17, la pupille 20 au milieu de l'iris 19, la chambre postérieure 18, ainsi qu'en traits pointillés, la capsule postérieure 21 réunie à l'extension du corps ciliaire 22 par des fibres zonulaires 23.

Comme cela apparaît sur la figure 4, la capsule postérieure 21 est tendue sur les indentations 8 et ce résultat a lui-même pour effet de retarder l'apparition des cataractes secondaires.

## Revendications

1. Implant intraoculaire de chambre postérieure, utilisable après ablation extracapsulaire du cristallin, comprenant une lentille (1) reposant par des anses souples (2) sur le sulcus ciliaire ou dans le sac cristallin et présentant au moins un trou de positionnement (6), la face antérieure de la lentille étant convexe et la face postérieure comportant des éléments distanceurs (8) éloignés du bord de la lentille et répartis de façon séparée entre eux sur cette face pour maintenir la capsule postérieure (21) tendue et éloignée de la face postérieure de la lentille, caractérisé en ce que les éléments distanceurs sont constitués par des pièces tronconiques (8) insérées dans des trous tronconiques (7) de la lentille (1), ces pièces (8) étant éloignées de 0,1 à 0,4 mm du bord de la lentille (1) et faisant saillie à l'extérieur de la face postérieure de la lentille sur une longueur de 0,3 à 0,4 mm mesurée aux endroits où sont percés les trous (7), de sorte que la distance entre la capsule postérieure (21) et la face postérieure soit au moins égale à 0,3 mm.

2. Implant selon la revendication 1, caractérisé en ce que les pièces tronconiques (8) sont disposées selon les diagonales d'un carré.

3. Implant selon l'une des revendications 1 ou 2, caractérisé en ce que l'anse supérieure (2) présente au voisinage de son extrémité supérieure une encoche (7a).

4. Implant selon l'une des revendications précédentes, caractérisé en ce que les pièces tronconiques (8) sont percées de trous longitudinaux (10).

## Claims

1. An intraocular implant for the posterior chamber, useable after extracapsular removal of the natural lens, the implant comprising a lens (1) resting against the ciliary sulcus or in the lens bag by means of flexible loops (2), and having at least one positioning hole (6), the front face of the lens being convex and the rear face including studs (8) set back from the edge of the lens and spaced apart on said face in order to maintain the posterior capsule (21) taut and at a distance from the rear face of the implant lens, characterized in that the studs are constituted by frustoconical pieces (8) inserted in frustoconical holes (7) in the implant lens (1), said pieces (8) being at a distance of 0.1 mm to 0.4 mm from the edge of the implant lens (1) and projecting from the rear face thereof over a distance of 0.3 mm to 0.4 mm measured at the locations where the holes (7) are formed, such that the distance between the posterior capsule (21) and the rear face is not less than 0.3 mm.

2. An implant according to claim 1, characterized in that the frustoconical pieces (8) are disposed on the diagonals of a square.

3. An implant according to claim 1 or 2, characterized in that the top loop (2) has a notch (7a) close to its top end.

4. An implant according to any preceding claim, characterized in that the frustoconical pieces (8) are pierced by longitudinal bores (10).

## Patentansprüche

1. Intra-okulares Hinterkammerimplantat, einsetzbar nach extra-kapsulärer Abtragung der Augenlinse, bestehend aus einer Linse (1) welche mittels weicher Schlingen (2) auf dem Ziliarsulkus oder im Linsensack gelagert ist und wenigstens ein Positionierungsöhr (6) enthält, wobei die Vorderseite der Linse konvex ist und die Hinterseite Abstandhalter (8) in einem gewissen Abstand vom Linsenrand enthält und voneinander getrennt auf dieser Seite verteilt sind, um die gespannte und von der Hinterseite der Linse entfernte Hinterkapsel zu halten, dadurch gekennzeichnet, dass die Abstandhalter aus in kegelstumpfartige Löcher (7) der Linse (1) eingelassenen kegelstumpfartigen Teilen (8) bestehen, wobei diese Teile (8) um 0,1 bis 0,4 mm vom Rand der Linse (1) entfernt sind und auf einer Länge von 0,3 bis 0,4 mm, gemessen an den Stellen wo die Löcher (7) eingestochen sind, aussen an der Hinterseite der Linse hervorstehen, so dass der Abstand zwischen der Hinterkapsel (21) und der Hinterseite mindestens gleich 0,3 mm ist.

2. Implantat nach Anspruch 1, dadurch gekennzeichnet, dass die kegelstumpfartigen Teile (8) in den Diagonalen eines Vierecks angeordnet sind.

3. Implantat nach einem der Ansprüche 1 oder 2, dadurch gekennzeichnet, dass die obere Schlinge (2) in der Nähe ihres oberen Endes eine Kerbe (7e) enthält.

4. Implantat nach einem der obigen Ansprüche, dadurch gekennzeichnet, dass die Kegelstumpfartigen Teile (8) mit Langlöchern (10) durchbohrt sind.

Fig:1

Fig:2

Fig:3

Fig. 4